# FASCICULE DE BREVET EUROPEEN

(11) **EP 3 014 158 B1**
(45) Date de publication et mention de la délivrance du brevet: **30.05.2018**
(21) Numéro de dépôt: 14750537.4
(22) Date de dépôt: 24.06.2014
(51) Int. Cl.: F16L 33/025, F16L 33/22, F16L 37/098, A61J 1/14, A61M 39/10, A61M 39/12

(54) **CONNECTEUR FLUIDIQUE AVEC COLLIER ET PROTECTION**
FLUIDSTECKER MIT KLEMME UND SCHUTZ
FLUID CONNECTOR WITH CLAMP AND PROTECTION

(30) Priorité: 28.06.2013 FR 1356350
(43) Date de publication de la demande: 04.05.2016
(73) Titulaire: SARTORIUS STEDIM FMT SAS, 13400 Aubagne (FR)
(72) Inventeur: BLAKE, Florian, F-83400 Hyeres (FR); GIBELIN, Jérémy, F-83330 Le Beausset (FR)
(74) Mandataire: Derambure Conseil
(86) Numéro de dépôt international: PCT/FR2014/051577
(87) Numéro de publication internationale: WO 2014/207368

(56) Documents cités:
- EP-A1- 2 497 989
- DE-A1- 10 347 927
- DE-A1-102005 038 439
- DE-C- 737 252
- FR-A1- 2 385 971
- FR-A1- 2 769 350
- US-A- 3 030 130
- US-A- 4 049 034
- US-A- 4 135 744
- US-A1- 2007 001 453

## Description

L'invention concerne les connecteurs fluidiques, en particulier pour les connexions ou raccordements fluidiques permettant de raccorder une conduite fluide à une autre conduite ou à un récipient, dans le domaine des applications biopharmaceutiques.

Plus précisément, les conduites ou tubes utilisées dans le domaine biopharmaceutique sont des tubes souples, voire très souples, qui servent à transporter des substances biopharmaceutiques diverses, avec le plus souvent des précautions d'asepsie nécessaires.

Dans les applications biopharmaceutiques, ce type de tube souple permet la circulation, le passage, la communication d'un fluide, tel qu'un fluide biopharmaceutique et peut être raccordé soit à un tube souple similaire soit à un récipient ou un contenant, qui peut être rigide ou souple lui aussi.

Le récipient ou contenant en question peut être en l'espèce un récipient de stockage et/ou de traitement d'un contenu tel qu'un produit biopharmaceutique. Un tel récipient s'entend en l'espèce d'un récipient rigide ou semi rigide réutilisable ou d'un récipient souple à usage unique tel qu'une poche, voire une cartouche filtrante.
Cette poche peut être une poche dite 2D, substantiellement peu épaisse, telle que celle commercialisée par Sartorius Stedim Biotech sous la marque Flexboy®, dont le volume, typiquement, peut être compris entre 50 millilitres et 50 litres. Cette poche souple peut aussi être une poche dite 3D, telle que celle commercialisée par Sartorius Stedim Biotech sous la marque Flexel®, avec un plus grand volume et une taille substantielle dans les trois dimensions. Il faut noter qu'un tube tel que celui auquel s'applique l'invention peut être interposé entre deux poches ou un plus grand nombre de poches.

Un tube tel que celui auquel s'applique l'invention, habituellement de section circulaire, est réalisé typiquement en matière plastique telle que le silicone, les élastomères thermoplastiques (TPE), mais également le PVC, la liste n'étant pas limitative. Il présente une certaine tenue d'ensemble et, simultanément, à la fois une certaine flexibilité d'ensemble et une certaine flexibilité locale, ce qui permet, moyennant l'exercice d'une force suffisante, de pincer le tube ou de le déformer substantiellement radialement.

Dans une réalisation typique, par exemple, le tube a un diamètre extérieur compris entre par exemple 8 millimètres et 30 millimètres, l'épaisseur dépendant du matériau, du diamètre et des applications.

Dans l'art connu, pour accoupler un tel tube souple, on enfile celui-ci sur un embout tubulaire, après quoi on place un collier de serrage autour du tube, puis on procède au serrage du collier. Ainsi, le collier serré exerce une pression radiale vers l'intérieur pour maintenir le tube souple sur l'embout, d'une part pour assurer une bonne étanchéité du tube vis-à-vis de l'embout et d'autre part pour éviter qu'une traction sur le tube ne conduise à un désengagement du tube de l'embout.

S'agissant de tels colliers de serrage, on peut utiliser par exemple un collier en matière plastique, de type polyamide par exemple en Rilsan®. Ce type de collier plastique, aussi parfois appelé Serflex®, comprend un système de crans sur une bande qui coopèrent avec un crochet à verrouillage agencé dans la tête, de manière à ce que le serrage ne soit pas réversible. En d'autres termes, une fois que l'on a engagé la bande dans la tête pour former une boucle, on tire sur la bande pour réduire le diamètre de la boucle et serrer le collier, le retour en arrière étant empêché par le crochet à verrouillage en prise dans un des crans de la bande. Après serrage, pour éviter que la bande ne dépasse trop du diamètre de la boucle du collier, on sectionne la portion libre de bande à proximité de la tête du collier. La partie restante non détachée de la bande présente souvent une arête vive qui peut s'avérer tranchante.

En alternative au collier plastique, on peut aussi utiliser un collier métallique qui se présente sous la forme d'un anneau préformé muni d'une ou deux oreilles faisant saillie vers l'extérieur relativement à la forme générale de l'anneau du collier, ce type de collier est parfois appelé collier Oetiker®. Après insertion du collier sur le tube à maintenir, on procède au moyen d'un outil au pincement de l'oreille (ou des oreilles) du collier ce qui provoque une déformation rémanente et ainsi un rétrécissement du diamètre principal de l'anneau et par conséquent serrage du collier sur le tube. Ce type de serrage par anneau métallique est particulièrement robuste et fiable.

Toutefois, à l'endroit de l'oreille pincée par l'outil, il peut se présenter une aspérité ou une bavure qui forme une arête vive qui peut s'avérer agressive.
Lorsque de tels colliers, qu'ils soient plastiques ou métalliques, sont installés dans des ensembles biopharmaceutiques, ces derniers peuvent être amenés à être transportés ou déplacés, et par conséquent il existe un risque d'agression par des parties agressives de ces colliers vis-à-vis d'autres éléments de l'ensemble biopharmaceutique, en particulier des poches souples ou des tubes souples, ce qui peut entraîner une fuite ou une perte de stérilisation préjudiciable à l'application biopharmaceutique.
De plus, ces colliers sont faciles d'accès (et donc peuvent être démontés) et ne permettent pas de garantir une image et une esthétique satisfaisantes.

Le document DE 103 47 927 A1 divulgue un dispositif de connexion fluidique comprenant un premier connecteur formant une interface mâle et un second connecteur formant une interface femelle. Les deux connecteurs sont couplés mutuellement dans une position de couplage relative par des moyens de clipsage.

Il existe par conséquent le besoin de proposer une amélioration destinée à pallier, au moins en partie, l'inconvénient précité de l'art antérieur connu.

Ci-après, il est divulgué un exposé de l'invention telle que caractérisée dans les revendications.
Selon un premier aspect, l'invention a pour objet un dispositif de connexion fluidique apte et destiné à raccorder une première paroi délimitant un premier espace fluidique, en forme de tube souple, à une seconde paroi délimitant un second espace fluidique, en forme de tube ou d'enceinte souple, de sorte à être apte et destiné à assurer une communication fluidique entre le premier espace fluidique et le second espace fluidique, comprenant :
- un premier connecteur délimitant un premier passage creux, apte et destiné à être raccordé à la première paroi et en communication fluidique avec le premier espace,
- un second connecteur délimitant un second passage creux, apte et destiné à être raccordé à la seconde paroi et en communication fluidique avec le second espace, le premier connecteur et le second connecteur étant en matière plastique et étant aptes et destinés à être couplés mutuellement dans une position de couplage relative, selon un axe A,
   le premier connecteur comprenant d'une part un embout tubulaire apte et destiné à être inséré dans une extrémité du tube et d'autre part une portion avant de couplage,
- un collier de serrage distinct, étant apte et destiné à être disposé autour de l'extrémité du tube pour serrer ledit tube sur l'embout tubulaire du premier connecteur,
le second connecteur comprenant des éléments de protection qui, dans la position de couplage, viennent se positionner au moins partiellement en vis-à-vis du collier de serrage dans la direction radiale, moyennant quoi le collier de serrage ne peut pas entrer en contact directement avec des éléments externes, les éléments de protection étant venus de matière à partir du corps du deuxième connecteur.
Moyennant quoi le collier de serrage ne peut pas entrer en contact directement avec des éléments externes on évite ainsi un endommagement possible des poches souples ou des tubes disposés au voisinage immédiat par une partie agressive du collier de serrage.
De plus, le corps du deuxième connecteur et les éléments de protection sont avantageusement obtenus intégralement par une seule opération de moulage économique.
Selon une réalisation, les éléments de protection sont reliés par des zones de charnière souple au corps du deuxième connecteur. Moyennant quoi, les éléments de protection ne constituent pas une gêne pendant l'insertion du premier connecteur ou du tube et du collier et peuvent être rabattus en position de protection dans un deuxième temps.
Selon une réalisation, lequel les éléments de protection sont des parois en forme de portion de cylindre. De sorte que la protection vis-à-vis de l'oreille du collier de serrage présente une forme optimisée, quelle que soit l'orientation du premier connecteur par rapport au second connecteur.
Selon une réalisation, les éléments de protection peuvent recouvrir le collier de serrage sur toute la circonférence et forment un anneau cylindrique de protection. Moyennant quoi, il est proposé une protection complète par rapport à l'oreille du collier de serrage quelle que soit sa position angulaire.
Selon une réalisation, le dispositif peut comprendre en outre une fonction anti-rotation pour empêcher les premier et deuxième connecteurs de tourner l'un par rapport à l'autre autour de l'axe A. De sorte que l'on peut éviter une rotation non désirée du premier connecteur avec son tube par rapport au second connecteur. On peut même en outre assurer une indexation du premier connecteur par rapport au second connecteur et permettre une insertion plus aisée pour l'opérateur en augmentant la zone de préhension.

Selon une réalisation, la fonction anti-rotation peut comprendre au moins un ergot agencé sur le premier connecteur, ledit ergot étant logé dans une encoche ménagée dans le deuxième connecteur. On peut ainsi proposer en outre une indexation du premier connecteur par rapport au second connecteur.

Selon une réalisation, le dispositif peut comprendre en outre un mécanisme de verrouillage de la position de couplage par des moyens de clipsage. Moyennant quoi on évite tout désaccouplement non désiré du dispositif de connexion après couplage, notamment lorsque des conditions de stérilité sont à respecter.

Selon une réalisation, les éléments de protection sont à distance de la surface extérieure du tube. Ceci permet de fournir un espace disponible pour l'oreille du collier de serrage. Ceci permet aussi une adaptation à différents diamètres de tube sur différents types de premier connecteur, pour un même type de second connecteur fixé sur poche souple ou autre tube.

Selon une réalisation, le collier de serrage est un collier métallique en forme générale d'anneau avec au moins une oreille, ladite oreille étant destinée à être pincée pour provoquer le serrage du collier. Ceci représente une solution standard bien maîtrisée pour la fonction collier de serrage.

Selon une réalisation, le premier connecteur forme une interface mâle apte à être reçue dans le second connecteur formant une interface femelle. Moyennant quoi le second connecteur équipé des éléments de protection peut être obtenu de façon relativement simple par moulage.

Selon une réalisation, le premier connecteur peut comprendre en outre une palette de préhension. Moyennant quoi on procure un élément sur lequel peuvent s'appuyer les doigts d'un utilisateur et cela facilite l'insertion du premier connecteur avec le tube assemblé dans le deuxième connecteur nonobstant la présence des éléments de protection.

Selon une réalisation, le deuxième connecteur peut comprendre en outre un identifiant de type code-barres ou étiquette RFID disposé sur les éléments de protection. Moyennant quoi l'accès à des informations relatives à la poche souple et/ou au produit biopharmaceutique y contenu est aisé, la position sur les éléments de protection étant particulièrement accessible et optimisée, ce qui facilite le processus de traçabilité.

Selon un deuxième aspect, l'invention a pour objet un ensemble biopharmaceutique comprenant un dispositif de connexion fluidique tel que décrit ci-dessus.

On propose ainsi un raccordement simple et sécurisé pour les ensembles biopharmaceutiques avec des tubes souples et des enceintes/poches. Avantageusement, les éléments de protection sont obtenus de matière avec le corps du raccord et l'embout, en une seule opération de moulage; moyennant quoi le raccord est peu coûteux bien qu'il intègre plusieurs fonctionnalités. Avantageusement, l'axe (W) des charnières souples est agencé sensiblement perpendiculairement à l'axe du raccord (A).

Selon une réalisation, les éléments de protection sont formés par deux parois hémi-cylindriques (4a, 4b) destinées à se refermer l'une vers l'autre en position de protection pour former une protection généralement cylindrique autour du collier de serrage ; moyennant quoi on dispose d'une solution simple à obtenir par une opération de moulage, solution performante du point de vue de la protection du collier.

Selon une réalisation, les éléments de protection de forme hémi-cylindriques sont maintenus en position de protection, une fois celle-ci atteinte, par un système de crochets ou de clipsage.
On décrit maintenant brièvement les figures des dessins.
La figure 1 est une vue éclatée du dispositif de connexion conforme à la présente invention.
La figure 2 est une vue en coupe axiale du dispositif de connexion de la figure 1, en position accouplée, selon la ligne de coupe II-II visible sur la figure 4.
La figure 3 est une coupe plus détaillée du dispositif de connexion de la figure 1, en position accouplée, selon la ligne de coupe III-III visible sur la figure 4.
La figure 4 est une coupe transversale détaillée du dispositif de connexion de la figure 1, en position accouplée, selon la ligne de coupe IV-IV visible sur la figure 3.
Les figures 5 et 5A représentent une variante de réalisation ne formant pas partie de l'invention dans laquelle les
éléments de protection du collier sont agencés sur le corps du deuxième connecteur au moyen de zones de charnière souples.
La figure 6 représente une variante de réalisation comprenant une fonction anti-rotation.
La figure 7 est analogue à la figure 5 et représente une autre variante de réalisation ne formant pas partie de l'invention.
Ci-après un exposé détaillé de plusieurs modes de réalisation de l'invention assorti d'exemples et de référence aux dessins.
Dans l'exemple illustré, il s'agit de raccorder tube souple 11 à un récipient 12 de produit biopharmaceutique au moyen d'un dispositif de connexion fluidique 10 qui comprend un premier connecteur 1 et un second connecteur 2.
Le tube souple 11 peut être défini généralement comme une première paroi 11 délimitant un premier espace fluidique 71.
Le récipient peut être défini généralement comme une enceinte souple 12 formée par une seconde paroi délimitant un second espace fluidique 72. Toutefois, ce qui est entendu par « seconde paroi » pourrait tout aussi bien définir un autre tube souple (non représenté aux figures).
Le **premier connecteur 1** comprend un embout tubulaire 9 à une de ses extrémités 1a, et une interface de couplage 6 avec le second connecteur à l'autre de ses extrémités. Le premier connecteur comprend en outre une portion intermédiaire 7 qui sera détaillée plus loin.
L'embout tubulaire 9 est de révolution autour de l'axe A, et il en est de même de l'interface de couplage 6. L'interface de couplage 6 est une interface de type mâle qui est prévue pour être insérée dans une interface de type femelle 20 décrite plus loin. Mais bien sûr, le contraire serait possible, c'est-à-dire une interface mâle dans le second connecteur et une interface femelle dans le premier connecteur, ou encore deux connecteurs asexués.

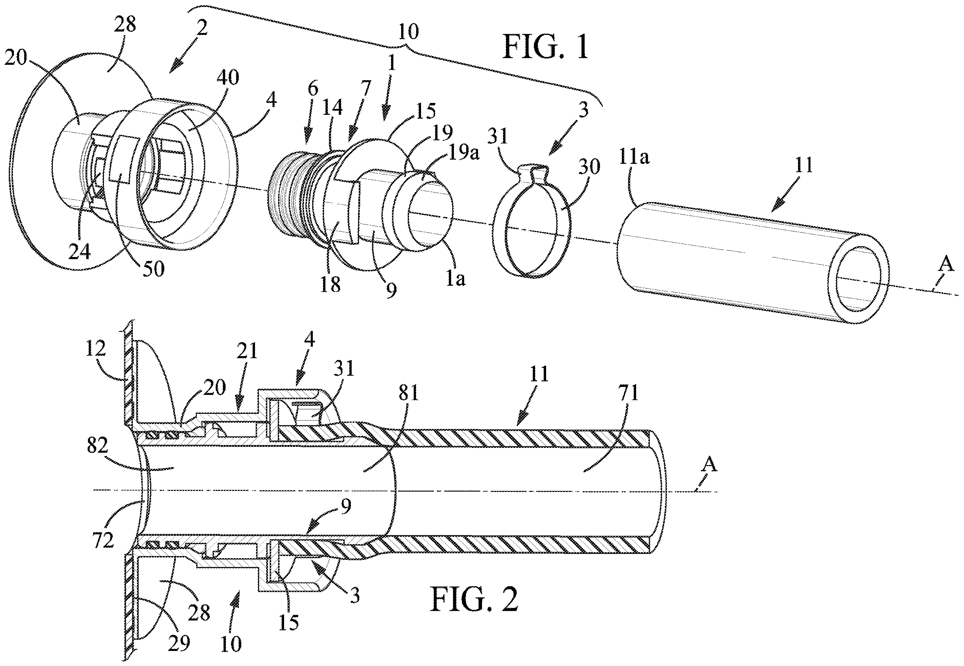

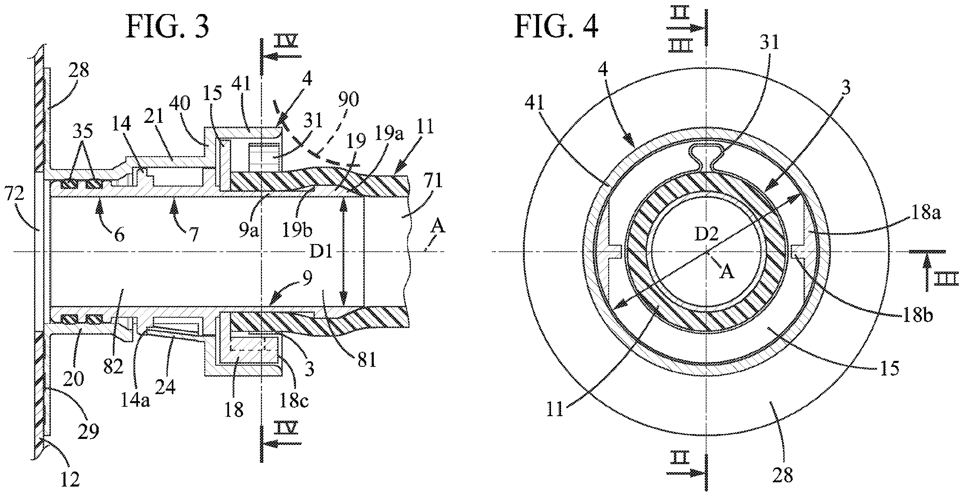

Dans l'exemple présenté, l'interface mâle 6 dans le premier connecteur est généralement cylindrique de révolution autour de A, avec une ou deux rainures extérieures destinées à recevoir un ou deux joints toriques 35 élastomères par exemple en silicone.
La portion intermédiaire 7 comprend une première collerette **14** en forme de disque dont l'utilité sera vue plus loin et une deuxième collerette **15** également en forme de disque, de plus grand diamètre, cette deuxième collerette sert de butée d'arrêt pour l'insertion du tube. La deuxième collerette 15 supporte aussi deux éléments de préhension aussi appelée « palettes » de préhension **18** qui servent à une manipulation aisée en vue de l'insertion du premier connecteur dans le second connecteur. Plus précisément chaque palette de préhension 18 comprend une portion de base 18a qui s'étend depuis la seconde collerette 15, une nervure de rigidification 18b et une face supérieure d'appui 18c sur laquelle peut venir s'appuyer l'extrémité d'un doigt.
L'embout tubulaire 9 comprend un bourrelet annulaire **19,** qui dans l'exemple illustré présente une rampe douce 19a du côté du tube souple à insérer et un épaulement 19b du côté opposé. L'embout tubulaire pourrait comprendre un nombre plus grand de bourrelets comme par exemple des crans successifs comme connu en soi.
On peut remarquer que le diamètre intérieur **D1** de l'embout tubulaire 9 est sensiblement voisin du diamètre intérieur du tube souple 11 au repos.
Lorsque l'on enfile le tube souple 11 sur l'embout tubulaire 9, le tube se déforme radialement vers l'extérieur grâce à la forme de rampe 19a, puis au fur et à mesure de l'insertion, il retrouve un diamètre plus étroit au niveau de la surface de portée 9a cylindrique.
L'insertion peut se poursuivre jusqu'à ce que l'extrémité avant du tube 11a vienne porter contre la deuxième collerette 15 susmentionnée (voir figures 2 et 3).

Une fois que le tube souple est inséré sur l'embout tubulaire 9, on vient placer au moins un **collier de serrage 3** autour du tube au niveau de la surface de portée **9a** susmentionnée. Il faut noter ici que le collier de serrage 3 peut être disposé préalablement en attente autour d'une partie arrière du tube avant l'opération d'insertion.
Une fois que le collier est dans la position adéquate en vis-à-vis de la surface de portée de l'embout tubulaire, on procède au serrage du collier.

Le collier de serrage représenté aux figures est un collier de type métallique avec une seule oreille **31** prévue pour le serrage. Toutefois il pourrait y avoir plus d'une oreille. L'oreille de serrage 31 est de préférence positionnée angulairement à distance de chacune des palettes de préhension 18 (cf Fig 4).

On utilise par exemple une pince pour venir écraser la forme en oreille 31 de manière à diminuer le diamètre de l'anneau 30 formé par le collier de serrage 3. De ce fait, le collier de serrage a alors un diamètre plus petit que celui de la surface extérieure du tube souple au repos, il exerce donc une pression radiale dirigée vers l'intérieur.
Cette pression radiale a deux objectifs : le premier est d'assurer étanchéité suffisamment performante entre le tube 11 et l'embout tubulaire 9, et le deuxième consiste à maintenir mécaniquement le tube autour de l'embout pour éviter qu'une traction exercée sur le tube ne conduise à désengager le tube de l'embout tubulaire, le deuxième objectif étant principalement atteint grâce à l'épaulement 19b susvisé.

Il faut noter qu'il est possible d'utiliser deux colliers côte à côte pour la fonction de serrage. Il faut aussi noter que des colliers métalliques sans oreille peuvent être utilisés (colliers à « rétreint serti »).

Le **second connecteur 2** comprend une collerette **28** de fixation à la poche souple 12, une telle fixation étant habituellement obtenue par une ou plusieurs soudures **29.** La collerette 28 est plane et vient se plaquer sur une surface importante sur la poche 12. Le centre de la collerette 28 est évidé pour établir une communication fluide entre l'espace cylindrique interne 82 du second connecteur et l'espace interne 72 de la poche.

De plus, le second connecteur 2 comprend une zone tubulaire **20** formant la partie femelle d'accouplement et recevant ainsi l'interface mâle 6 susmentionnée ; en position accouplée, les joints toriques 35 susmentionnés sont pressés vers l'intérieur par la surface cylindrique intérieure de la zone tubulaire 20 formant l'interface femelle. On obtient ainsi un raccordement fluide étanche entre le premier connecteur 1 et le second connecteur 2.
De plus, le second connecteur 2 comprend un **corps 21** qui s'étend depuis la zone tubulaire 20 dans une direction opposée à la collerette 28.

Le corps 21 se présente comme une forme de cloche d'axe A avec d'une part une portion médiane comprenant des languettes de verrouillage **24,** ici au nombre de deux bien que leur nombre puisse être quelconque, et d'autre part au voisinage de l'extrémité ouverte, une portion cylindrique **41** de diamètre plus large **D2** qui forme des éléments de protection **4** dont la fonction sera détaillée ci-dessous.
Comme visible sur les figures 2 et 3, la deuxième collerette 15 est reçue à l'intérieur de la portion cylindrique large de diamètre **D2,** jusqu'au fond **40** de cette dernière en position de couplage, on obtient ainsi une butée de fin de course du mouvement d'insertion du premier connecteur dans le second connecteur.
La jante extérieure **41** de la portion cylindrique large de diamètre D2 forme ce qui est appelé « des éléments de protection » vis-à-vis du collier de serrage 3 : en effet celui-ci, en position de couplage, se retrouve dans la zone intérieure définie par cette portion cylindrique 41. Moyennant quoi, si lors des manipulations ou déplacements du dispositif de connexion, ce dernier peut venir contacter des éléments externes **90** alors ce n'est pas le collier de serrage qui viendra contacter lesdits éléments externes 90 mais au lieu de cela ce seront les éléments de protection 4 ici en l'occurrence jante extérieure 41 de la portion cylindrique qui viendra contacter le ou les éléments externes 90 (voir figure 3).
Ainsi, tout endommagement par contact avec une partie agressive du collier 3 peut être avantageusement évité.
Il faut bien noter qu'on peut entendre par éléments de protection toute forme qui peut être interposée entre le collier de serrage 3 et des éléments externes 90. En particulier, il n'est pas nécessaire que la protection forme un anneau continu comme dans l'exemple représenté, il pourrait s'agir d'une pluralité de pattes disjointes réparties sur la circonférence, ou encore une pluralité de parois en forme de portions cylindriques, disjointes et réparties sur la circonférence.
Avantageusement, ces éléments de protection sont obtenus intégralement par moulage du second connecteur, c'est-à-dire des éléments de protection sont venus de matière vis-à-vis du corps 21 du second connecteur 2.

Les premier et second connecteurs 1,2 peuvent être obtenus par moulage d'une matière plastique, par exemple polypropylène, du polyéthylène, du polycarbonate, du polysulfone.

Selon un aspect optionnel, il peut être prévu des languettes de verrouillage **24** dans le corps 20 du second connecteur. Ces languettes de verrouillage 24, au nombre de deux dans l'exemple illustré sont flexibles, elles s'écartent vers l'extérieur au moment où la première collerette 14 avance au niveau de leur extrémité libre, puis reviennent vers l'intérieur pour se positionner en face d'une butée anti retrait 14a sur l'arrière de la première collerette 14. Après quoi il n'est pas possible de faire marche arrière c'est-à-dire de retirer le premier connecteur du second connecteur sans au préalable effacer d'une façon ou d'une autre les languettes de verrouillage 24.

Les matières des premier et second connecteurs peuvent être de couleurs différentes ce qui permet de facilement vérifier de visu la bonne position de la languette de verrouillage 24 contre la première collerette 14.

Il faut remarquer que le premier connecteur 1 délimite un premier passage creux **81** destiné à être mis en communication fluidique avec le premier espace fluide **71.** De même, le second connecteur délimite le second passage creux **82** déjà évoqué, destiné à être mis en communication fluidique avec le second espace fluide **72** (l'intérieur de la poche plastique dans le cas particulier illustré).

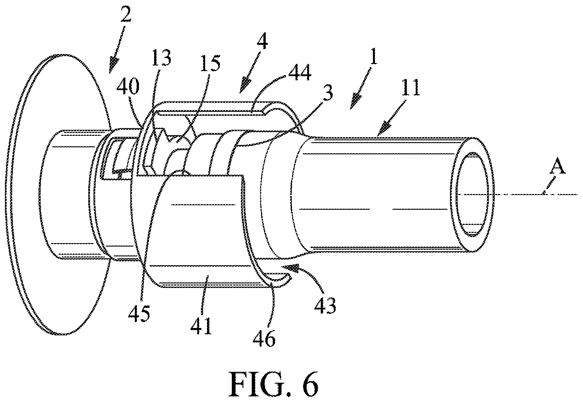

Selon une caractéristique optionnelle, illustrée à la figure 6, le dispositif de connexion **10** peut comprendre une fonction anti rotation pour empêcher le premier connecteur **1** de tourner par rapport au second connecteur **2** autour de l'axe A. Plus précisément, il s'agit par exemple d'un ergot radial **13** agencé sur le premier connecteur par exemple au niveau de la deuxième collerette **15.** Cet ergot 13 est reçu dans une encoche **43** ménagée dans la jante **41** de la forme en cloche 4 qui forme des éléments de protection du collier. Dans l'exemple illustré, il y a deux ergots 13 diamétralement opposés reçus chacun respectivement dans une encoche **43.** Chaque encoche **43** est délimitée par une première bordure **44** axiale et une seconde bordure **45** axiale qui s'étendent chacune du fond 40 jusqu'à l'extrémité **46** avant des éléments de protection 4.

Cette configuration permet de faciliter le mouvement d'insertion du premier connecteur par un utilisateur qui le saisit au niveau des ergots ; il peut pousser les ergots 13 dans les encoches 43 précitées juste à ce que la première collerette 15 soit au contact du fond 40 radial des éléments de protection. Cette configuration permet aussi de limiter la rotation du premier connecteur 1 à l'intérieur du deuxième connecteur 2.

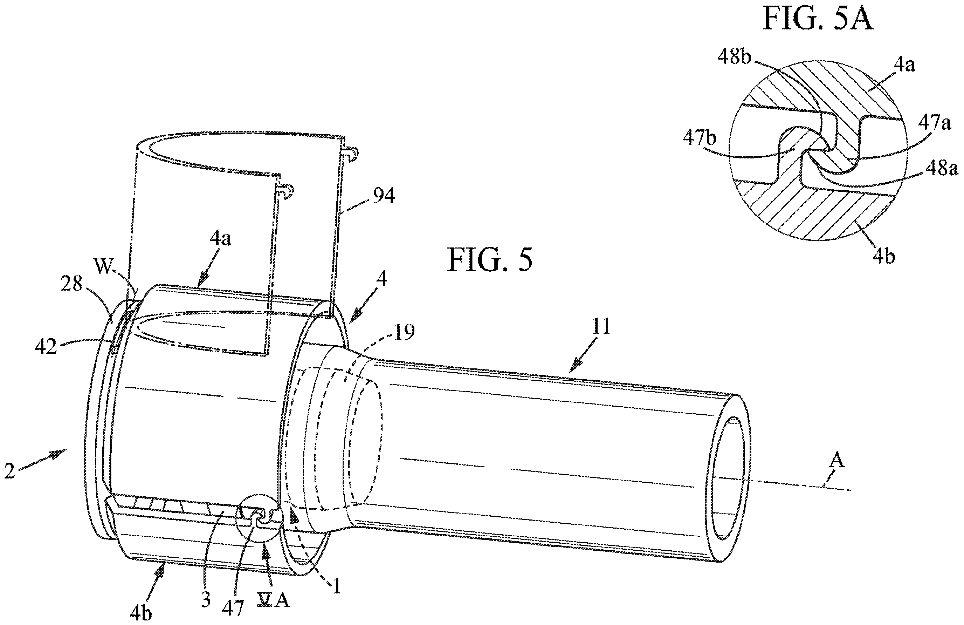

Dans une variante de réalisation ne formant pas partie de l'invention représentée à la figure 5, les éléments de protection 4 ne sont pas formés de manière rigide par rapport au corps 21 du second connecteur mais ils sont reliés à ce dernier par des zones de charnière souples 42. Par exemple, il peut être prévu deux demi-coquilles **4a, 4b,** ouvertes en situation initiale pour ne pas gêner l'insertion (seule la demi-coquille 4a est représentée dans cette situation initiale, en trait mixte **94,** sur la figure 5) ; après insertion, lesdites deux demi-coquilles sont rabattues l'une vers l'autre par pivotement (la demi-coquille 4a tourne autour de l'axe **W**), pour venir se clipser l'une à l'autre au moyen de formes de verrouillage **47.** Les crochets peuvent se présenter comme des crochets élastiques complémentaires référencés **47a,47b** sur la Figure 5A ; les rampes 48a,48b complémentaires conjuguées avec l'élasticité des tiges de crochets permettent un verrouillage automatique à la fermeture.
Toute autre forme de crochets ou de moyen de clipsage équivalent pourrait tout aussi bien être utilisée pour maintenir les deux demi-coquilles l'une près de l'autre dans une position où elles forment ensemble des éléments de protection 4 pour empêcher que des éléments externes 90 ne viennent au contact directement du collier 3.

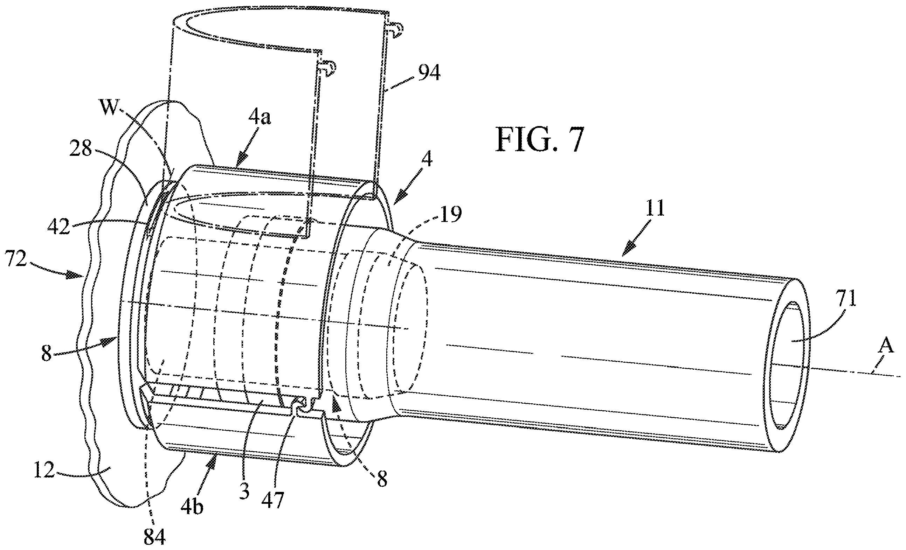

Selon une variante ne formant pas partie de l'invention représentée à la figure 7, le premier connecteur 1 et le second connecteur 2 sont formés comme une seule et même pièce 8, dans un contexte où le tube souple **11** est raccordé de manière quasi définitive (usage unique) sur une poche ou enceinte **12,** le tube souple et la poche/enceinte étant identiques ou similaires à ce qui a été décrit précédemment. Le collier de serrage **3** est similaire à celui décrit plus haut dans sa forme et ses fonctions.
Le raccord **8** se présente comme une pièce plastique intégralement obtenue de moulage, le raccord comprenant un corps **84,** un embout tubulaire **19** similaire ou identique à celui décrit précédemment, et une collerette **28** de fixation à la poche, similaire ou identique à ce qui a été décrit ci-dessus en référence aux figures 1-6 .
De plus, le raccord comprend des éléments de protection **4a,4b** reliés au corps **84** du raccord par des zones de charnière souple **42.** Dans l'exemple illustré, les éléments de protection sont formés par deux parois hémi-cylindriques destinées à se refermer l'une vers l'autre en position de protection pour former une protection généralement cylindrique autour du collier de serrage, comme cela a déjà été décrit en référence à la figure 5.
Avantageusement, les éléments de protection sont déplaçables entre d'une part une **position d'attente** (en trait mixte **94,** sur la figure 7) facilitant l'insertion (manuel ou automatique) du tube sur l'embout et d'autre part une **position de protection** (en trait plein sur la figure 7) dans laquelle les éléments de protection viennent se positionner autour du collier de serrage **3.**
Avantageusement, l'axe **W** des charnières souples est agencé sensiblement perpendiculairement à l'axe du raccord **A.**
Avantageusement, les éléments de protection de forme hémi-cylindriques sont maintenus en position de protection, une fois celle-ci atteinte, par un système de crochets ou de clipsage **47** déjà commentés plus haut dans les commentaires de la figure 5A.
De préférence, les éléments de protection **4a,4b** se trouvent par défaut en position d'attente, et le déplacement vers la position de protection nécessite de vaincre une force de rappel élastique modérée. Ainsi, on peut insérer directement le tube sans précaution préalable ou préparation, et sans gêne des éléments de protection. Dans un deuxième temps, on referme ensuite l'un vers l'autre les éléments de protection qui s'accrochent automatiquement l'un à l'autre pour former une protection effective du collier.

En outre, il est prévu une caractéristique optionnelle compatible avec toutes les variantes de réalisation précédemment évoquées : il s'agit de l'intégration d'un identifiant 50, de type code barre ou étiquette électronique par exemple de type RFID.On dispose cet identifiant dans le premier connecteur 1 ou le second connecteur 2, de manière plus préférée au niveau des éléments de protection 4 qui sont faciles d'accès depuis l'extérieur. On peut ainsi procéder facilement à une lecture des données au moyen d'un outil de lecture.

Dans le cas d'une étiquette RFID, celle-ci peut être positionnée dans n'importe quel endroit, à savoir le premier connecteur ou le second connecteur, sans nécessité d'axe visible.

## Revendications

1. Dispositif de connexion fluidique (10) apte et destiné à raccorder une première paroi (11) délimitant un premier espace fluidique (71), en forme de tube souple (11), à une seconde paroi délimitant un second espace fluidique (72), en forme de tube ou d'enceinte souple ou rigide à usage unique, dans un ensemble biopharmaceutique, de sorte à être apte et destiné à assurer une communication fluidique entre le premier espace fluidique et le second espace fluidique, comprenant
- un premier connecteur (1) délimitant un premier passage creux (81), apte et destiné à être raccordé à la première paroi et en communication fluidique avec le premier espace,
- un second connecteur (2) délimitant un second passage creux (82), apte et destiné à être raccordé à la seconde paroi et en communication fluidique avec le second espace,
le premier connecteur et le second connecteur étant en matière plastique et étant aptes et destinés à être couplés mutuellement dans une position de couplage relative, selon un axe A,
le premier connecteur comprenant d'une part un embout tubulaire (9) apte et destiné à être inséré dans une extrémité du tube et d'autre part une portion avant de couplage,
- au moins un collier de serrage (3) distinct, étant apte et destiné à être disposé autour de l'extrémité du tube (11) pour serrer ledit tube sur l'embout tubulaire du premier connecteur,
le second connecteur comprenant des éléments de protection (4) qui, dans la position de couplage, viennent se positionner au moins partiellement en vis-à-vis du collier de serrage dans la direction radiale, moyennant quoi le collier de serrage ne peut pas entrer en contact directement avec des éléments externes,
dans lequel les éléments de protection (4) sont venus de matière à partir du corps (21) du deuxième connecteur.

2. Dispositif selon la revendication 1, dans lequel les éléments de protection (4) sont reliés par des zones de charnière souple (42) au corps du deuxième connecteur.

3. Dispositif selon l'une des revendications 1-2, dans lequel les éléments de protection sont des parois en forme de portion de cylindre.

4. Dispositif selon l'une des revendications 1-3, dans lequel les éléments de protection recouvrent le collier de serrage sur toute la circonférence et forment un anneau cylindrique de protection.

5. Dispositif selon l'une des revendications 1-4, comprenant en outre une fonction anti-rotation pour empêcher les premier et deuxième connecteurs de tourner l'un par rapport à l'autre autour de l'axe A.

6. Dispositif selon la revendication 5, dans lequel la fonction anti-rotation comprend au moins un ergot agencé sur le premier connecteur, ledit ergot étant logé dans une encoche ménagée dans le deuxième connecteur.

7. Dispositif selon l'une des revendications 1-6, comprenant en outre un mécanisme de verrouillage de la position de couplage par des moyens de clipsage.

8. Dispositif selon l'une des revendications 1-7, dans lequel les éléments de protection (4) sont à distance de la surface extérieure du tube (11).

9. Dispositif selon l'une des revendications 1-8, dans lequel le collier de serrage est un collier métallique en forme générale d'anneau avec au moins une oreille, ladite oreille étant destinée à être pincée pour provoquer le serrage du collier.

10. Dispositif selon l'une des revendications 1-9, dans lequel le premier connecteur forme une interface mâle apte à être reçue dans le second connecteur (2) formant une interface femelle.

11. Dispositif selon l'une des revendications 1 à 10, dans lequel le premier connecteur comprend en outre une palette de préhension (18).

12. Dispositif selon l'une des revendications 1-11, dans lequel le deuxième connecteur comprend en outre un identifiant (50) de type code barre ou étiquette RFID ou code-barres dosposé sur les éléments de protection.

13. Ensemble biopharmaceutique comprenant un dispositif de connexion fluidique selon l'une des revendications 1 à 12.

## Patentansprüche

1. Fluidverbindungs-Vorrichtung (10), welche dazu eingerichtet und vorgesehen ist, eine erste Wand (11), welche einen ersten Fluidraum (71) in Form eines elastischen Rohrs (11) begrenzt, mit einer zweiten Wand, welche einen zweiten Fluidraum (72) in Form eines Rohrs oder Behälters zur einmaligen Verwendung, welches/welcher elastisch oder steif ist, in einer biopharmazeutischen Anordnung derart zu verbinden, dass sie dazu eingerichtet und vorgesehen ist, eine Fluidverbindung zwischen dem ersten Fluidraum und dem zweiten Fluidraum sicherzustellen, umfassend:
- ein erstes Verbindungselement (1), welches einen ersten hohlen Durchgang (81) begrenzt, welcher dazu eingerichtet und vorgesehen ist, an der ersten Wand und in Fluidverbindung mit dem ersten Raum verbunden zu sein,
- ein zweites Verbindungselement (2), welches einen zweiten hohlen Durchgang (82) begrenzt, welcher dazu eingerichtet und vorgesehen ist, an der zweiten Wand und in Fluidverbindung mit dem zweiten Raum verbunden zu sein,
wobei das erste Verbindungselement und das zweite Verbindungselement aus Kunststoff bestehen und dazu eingerichtet und vorgesehen sind, miteinander in einer relativen Kopplungsposition entlang einer Achse A gekoppelt zu sein, wobei das erste Verbindungselement einerseits ein rohrförmiges Endstück (9), welches dazu eingerichtet und vorgesehen ist, in ein Ende des Rohrs eingesetzt zu sein, und andererseits einen vorderen Kopplungsteil umfasst,
- wenigstens eine separate Klemmschelle (3), welche dazu eingerichtet und vorgesehen ist, um das Ende des Rohrs (11) herum angeordnet zu sein, um das Rohr auf dem rohrförmigen Endstück des ersten Verbindungselements zu verklemmen,
wobei das zweite Verbindungselement Schutzelemente (4) umfasst, welche in der Kopplungsposition wenigstens teilweise gegenüber der Klemmschelle in der radialen Richtung in Position kommen, wodurch die Klemmschelle nicht in direkten Kontakt mit externen Elementen kommen kann,
wobei die Schutzelemente (4) ausgehend von dem Körper (21) des zweiten Verbindungselements einteilig gebildet sind.

2. Vorrichtung nach Anspruch 1, wobei die Schutzelemente (4) durch elastische Scharnierbereiche (42) an dem Körper des zweiten Verbindungselements angebracht sind.

3. Vorrichtung nach einem der Ansprüche 1-2, wobei die Schutzelemente Wände in Form von Zylinderabschnitten sind.

4. Vorrichtung nach einem der Ansprüche 1-3, wobei die Schutzelemente die Klemmschelle auf dem gesamten Umfang bedecken und einen zylindrischen Schutzring bilden.

5. Vorrichtung nach einem der Ansprüche 1-4, ferner umfassend eine Anti-Rotations-Funktion zum Verhindern, dass sich das erste und zweite Verbindungselement gegeneinander um die Achse A verdrehen.

6. Vorrichtung nach Anspruch 5, wobei die Anti-Rotations-Funktion wenigstens einen an dem ersten Verbindungselement angeordneten Nocken umfasst, wobei der Nocken in einer an dem zweiten Verbindungselement vorgesehenen Nut aufgenommen ist.

7. Vorrichtung nach einem der Ansprüche 1-6, ferner umfassend einen Verriegelungsmechanismus für die Kopplungsposition mittels Schnappmitteln.

8. Vorrichtung nach einem der Ansprüche 1-7, wobei die Schutzelemente (4) in einem Abstand von der Außenfläche des Rohrs (11) liegen.

9. Vorrichtung nach einem der Ansprüche 1-8, wobei die Klemmschelle eine metallische Schelle im Wesentlichen in Form eines Rings mit wenigstens einem Ohr ist, wobei das Ohr dazu vorgesehen ist, zusammengepresst zu werden, um das Klemmen der Schelle hervorzurufen.

10. Vorrichtung nach einem der Ansprüche 1-9, wobei das erste Verbindungselement eine männliche Schnittstelle bildet, welche dazu eingerichtet ist, in dem zweiten Verbindungselement (2) aufgenommen zu sein, welches eine weibliche Schnittstelle bildet.

11. Vorrichtung nach einem der Ansprüche 1 bis 10, wobei das erste Verbindungselement ferner eine Greiflasche (18) umfasst.

12. Vorrichtung nach einem der Ansprüche 1 bis 11, wobei das zweite Verbindungselement ferner eine Kennung (50) vom Barcode-Typ oder einen RFID- oder Barcode-Chip umfasst, welcher an den Schutzelementen angeordnet ist.

13. Biopharmazeutische Anordnung, umfassend eine Fluidverbindungs-Vorrichtung nach einem der Ansprüche 1 bis 12.

## Claims

1. Fluid-connection device (10) adapted and intended for connecting a first wall (11) defining a first fluid space (71), in the form of a flexible pipe (11), to a second wall defining a second fluid space (72), in the form of a pipe or an enclosure that is flexible or rigid and disposable, in a biopharmaceutical assembly, for ensuring fluid communication between the first fluid space and the second fluid space, comprising:
- a first connector (1) defining a first hollow passage (81), adapted and intended for connection to the first wall and in fluid communication with the first space,
- a second connector (2) defining a second hollow passage (82), adapted and intended for connection to the second wall and in fluid communication with the second space,
the first connector and the second connector being of plastic and being adapted and intended to be coupled together in a relative coupling position, along an axis A,
the first connector comprising a tubular nozzle (9) adapted and intended for insertion into one end of the pipe, and a front coupling portion,
- at least one separate pipe clamp (3), adapted and intended for placement around the end of the pipe (11) in order to clamp said pipe onto the tubular nozzle of the first connector,
the second connector comprising protective elements (4) which, in the coupling position, are positioned so as to at least partially face the pipe clamp in the radial direction, whereby the clamp cannot come into direct contact with external elements, wherein the protective elements (4) are integrally formed from the body (21) of the second connector.

2. Device according to claim 1, wherein the protective elements (4) are connected by flexible hinge areas (42) to the body of the second connector.

3. Device according to one of claims 1-2, wherein the protective elements are walls in the shape of cylindrical portions.

4. Device according to one of claims 1-3, wherein the protective elements overlie the pipe clamp along its entire circumference and form a cylindrical protective ring.

5. Device according to one of claims 1-4, further comprising an anti-rotation feature to prevent the first and second connectors from rotating relative to one another about the axis A.

6. Device according to claim 5, wherein the anti-rotation feature comprises at least one lug arranged on the first connector, said lug being received in a notch formed in the second connector.

7. Device according to one of claims 1-6, further comprising a mechanism for locking the coupling position by snap-fitting means.

8. Device according to one of claims 1-7, wherein the protective elements (4) are at a distance from the outer surface of the pipe (11).

9. Device according to one of claims 1-8, wherein the pipe clamp is a metal clamp having the general shape of a ring with at least one ear, said ear being intended to be crimped to tighten the clamp.

10. Device according to one of claims 1-9, wherein the first connector forms a male interface adapted to be received in the second connector (2) forming a female interface.

11. Device according to one of claims 1-10, wherein the first connector further comprises a gripping tab (18).

12. Device according to one of claims 1-11, wherein the second connector further comprises an identifier (50) such as a barcode or RFID tag placed on the protective elements.

13. Biopharmaceutical assembly comprising a fluid-connection device according to one of claims 1 to 12.
